# EUROPÄISCHE PATENTSCHRIFT

(11) **EP 1 621 182 B1**
(45) Veröffentlichungstag und Bekanntmachung des Hinweises auf die Patenterteilung: **20.06.2007**
(21) Anmeldenummer: 05004498.1
(22) Anmeldetag: 02.03.2005
(51) Int. Cl.: A61K 8/36, A61Q 1/10, C09D 13/00

(54) **Kosmetikmine**
Cosmetic pencil
Crayon cosmétique

(30) Priorität: 23.07.2004 DE 202004011608 U; 12.08.2004 DE 202004012652 U
(43) Veröffentlichungstag der Anmeldung: 01.02.2006
(73) Patentinhaber: Faber-Castell AG, 90546 Stein (DE)
(72) Erfinder: Appel, Reiner, 90522 Oberasbach (DE); Lugert, Gerhard, Dr., 90431 Nürnberg (DE); von Godin, Harald, 90522 Oberasbach (DE); Kinzel, Joachim, 90547 Stein (DE)
(74) Vertreter: Mörtel & Höfner

(56) Entgegenhaltungen:
- EP-A- 1 258 235
- EP-A- 1 291 399
- DE-C1- 3 437 989

## Beschreibung

Kosmetische Minen für dekorative und auch hautpflegende Zwecke werden bei Lippenstiften, Lipliner, Eyeshadow-Produkten, Eyeliner, Kajal-Stiften, Rouge-Stiften und dergleichen verwendet. Wenn hier von Minen gesprochen wird, so sind darunter auch Kreiden zu verstehen. Sie enthalten im Allgemeinen eine Minengrundmasse aus wachsartigen Substanzen wie Wachsen, Fetten und Emulgatoren sowie Ölen, Füllstoffen und Pigmenten.

Nachteilig bei derartigen Minen ist, dass sie eine relativ geringe Temperaturstabilität und insbesondere eine geringe maximale Applikationstemperatur aufweisen. Als maximale Applikationstemperatur ist dabei die Temperatur zu verstehen, bei welcher ein Aufbringen der Minen- oder Kreidenmasse auf die Lippe, das Augenlid oder andere Hautpartien ohne Verschmieren, Verformungen der Mine oder Abbrechen der Mine bzw. Kreide gerade noch möglich ist. Herkömmliche Minen weisen zwar Schmelzpunkte bzw. Schmelzbereiche zwischen 60 °C und 90°C auf, ihre maximale Applikationstemperatur ist aber naturgemäß weit geringer. Die zur Ausformung der Mine angewandten Verfahren beeinflussen die Mikrostruktur der Minengrundmasse und damit die mechanischen und Applikationseigenschaften der späteren Mine. Am geringsten ist die Beeinflussung beim Gießverfahren, mit dem sich Minen mit Applikationstemperaturen von etwa 40°C bis 45°C erzeugen lassen. Beim Strangpressen, bei dem die Minen-Ausgangsmasse in einem Zylinder komprimiert wird und diesen über eine Austrittsöffnung in Strangform verlässt, wird die Minengrundmasse mechanisch und auch thermisch derart belastet, dass die fertigen Minen Applikationstemperaturen nur von etwa 30 °C bis 35 °C aufweisen.

EP-1 291 399 und EP-1 258 235 offenbaren Kosmetikminen, die einen Füllstoff und einen Verdicker enthalten.

Davon ausgehend ist es die Aufgabe der Erfindung, eine Mine für kosmetische Anwendungen vorzuschlagen, die hinsichtlich ihrer Temperaturstabilität verbessert ist, die insbesondere bei höheren Temperaturen applizierbar ist.

Diese Aufgabe wird durch eine Mine nach Anspruch 1 gelöst. Danach ist vorgesehen, dass die Minengrundmasse der Mine von wenigstens einem Fettsäuresalz und wenigstens einer lipophilen Substanz mit einem Schmelzbereich < 50 °C, d.h. einem Öl oder einer wachsartigen Substanz, gebildet ist. Unter Minengrundmasse ist dabei jene Masse zu verstehen, die als Bindemittel für die Feststoffpartikel dient und die für die Applikationseigenschaft und die thermische Stabilität der Mine maßgeblich verantwortlich ist.

Überraschenderweise hat sich gezeigt, dass mit derartigen Grundmassen Minen herstellbar sind, die Schmelzpunkte von über 90 °C haben, also weit höhere Lagertemperaturen schadlos überstehen als herkömmliche Minen und bei höheren Temperaturen als diese, nämlich bei mehr als 50 °C applizierbar sind. Weiterhin ist überraschend, dass Minen mit der vorgeschlagenen Minengrundmasse bis zu 90 % Feststoffpartikel aufnehmen können, wodurch sehr farbintensive Minenzubereitungen herstellbar sind. Mit herkömmlichen Minengrundmassen sind derartige Minen nicht herstellbar. Aufgrund des hohen Anteils an Feststoffpartikeln hätte eine in den schmelzflüssigen Zustand gebrachte Minenmasse eine zu hohe Viskosität, als dass sie sich noch vergießen ließe. Aber auch eine Ausformung im Strangpressverfahren oder durch eine Extrusion etwa mittels eines Schneckenextruders wäre nicht möglich, da sich herkömmliche Minengrundmassen bei den erforderlichen Temperaturen zu stark verflüssigen würden. Eine erfindungsgemäße Minenmasse lässt sich dagegen problemlos bei Temperaturen von etwa 80 °C bis etwa 140 °C extrudieren, u.a. weil die Minenmasse bzw. Minengrundmasse selbst bei diesen Temperaturen noch eine ausreichend hohe Viskosität bei der Verarbeitung aufweist. Besonders gute Ergebnisse werden mit Minen erzielt, die 1 % bis 20 %, vorzugsweise 3 % bis 10 % Fettsäuresalz enthalten. Die besten Ergebnisse werden mit Fettsäuregehalten von 4 % bis 8 % erzielt. Als Anteil für eine lipophile Substanz sind 5 % bis 40, vorzugsweise 10 % bis 35 % vorgesehen. Als besonders geeignet hat sich ein Gehalt an lipophiler Substanz von 16 % bis 32 % herausgestellt.

Günstig auf die Temperaturstabilität der Minen und insbesondere auf ihre Applikationstemperatur wirkt sich ein Gehalt an Feststoffpartikeln von mindestens 50 %, besser noch von 60 % bis 90 % aus. Unter Feststoffpartikel sind dabei Füllstoffe wie Talkum und Glimmer aber auch beispielsweise Farbpigmente zu verstehen. Die Kosmetikminen setzen sich aus 1 - 20 % Fettsäuresalz, 5 - 40 % lipophile Substanz, 50 - 90 % Feststoffpartikel, 0 - 10 % Additive zusammen, bevorzugte Minenvarianten bestehen aus 4 - 8 % Fettsäuresalz, 16 - 32 % lipophile Substanz, 60 - 80 % Feststoffpartikel, 0 - 10 % Additive.

Als geeignete Fettsäuresalze sind solche mit Kettenlängen von 10 bis 20 C -Atomen, insbesondere, Calciumlauriat, Magnesiumstearat, Zinkstearat, aber auch Palmitate, Myristate, Oleate und Linoleate zu nennen. Besonders geeignet sind Lithiumstearat, Aluminiumstearat, Aluminiumtristearat sowie Mischungen der genannten Fettsäuresalze. Optimale Ergebnisse werden mit Aluminiumdistearat erreicht.

Als lipophile Flüssigkeiten bzw. Öle oder wachsartige Substanzen mit niedrigem Schmelzpunkt < 50°C können Castoröl, Mineralöl, Stearyl-dimethicon, hydrogeniertes (gehärtetes) Kokosöl, Isopropylpalmitat, Isopropylmyristat, Triglyceride wie Capryl- und Caprin-Triglycerid, Hydrogenierte Palmkernöl-Glyceride, und Hydrogenierte Palmöl-Glyceride aber auch Olivenöl, Rapsöl sowie andere für Kosmetika übliche Öle Verwendung finden.

Als Farbmittel können für Kosmetik übliche organische oder anorganische Pigmente zugesetzt werden. Zusätzlich ist der Einsatz von Farbstoffen, etwa lipophilen Farbstoffen denkbar. Bei Bedarf kann die Zugabe von Füllstoffen wie z. B. Talkum, Kaolin, Glimmer oder mit Siliciumdioxid beschichteter Glimmer, denkbar. Als Additive bzw. Hilfsstoffe können bei Bedarf Konservierungsmittel, Sonnenschutzmittel, Aromen, Vitamine, Parfums, pflegende Substanzen, aber auch geringe Anteile von Struktur- bzw. Texturbildnern wie C 30-45 Alkyl Methicone (CAS Nr. 131459-42-2) zugesetzt sein. Die Summe der Additive beträgt hierbei maximal 10 % innerhalb der Formulierung.

Die erfindungsgemäßen Massen werden in Mischern, Knetern, Walzenstühlen o. ä. homogenisiert und bei 80-140°C zu Minen bzw. Kreiden extrudiert.

Als Basisrezeptur kann die folgende Zusammensetzung angegeben werden:

| | |
|---|---|
| Salz einer Fettsäure: | 1 - 20 % |
| Lipophile Substanz mit Schmelzpunkt < 50.°C | 5 - 40 % |
| Pigmente, Füllstoffe: | 50 - 90 % |
| Additive: | 0 - 10 % |

Eine bevorzugte Basisrezeptur weist die folgende Zusammensetzung auf:

| | |
|---|---|
| Fettsäuresalz: | 4 - 8 % |
| Lipophile Substanz | 16 - 32 % |
| Feststoffpartikel: | 60 - 80 % |
| Additive: | 0 - 10 % |

Durch Beispiele soll eine Konkretisierung der Erfindung erfolgen:

### Beispiel 1:

### Blaue Eyeliner-Mine im Minendurchmesser 4 mm:

| | |
|---|---|
| Lithiumstearat: (CAS 4485-12-5) | 8,0 % |
| Isopropylmyristat:(CAS 110-27-0) | 20,0 % |
| Hydrogenated Palmkernelglycerides (Handelsname Lipocire CM; Bezug bei: Gattefossé): | 11,0 % |
| Pigment Blau 27; CI 77510 | 60,0 % |
| Pantenol (CAS 16485-10-2): | 0,5 % |
| Propylparaben: | 0,5 % |

### Beispiel 2:

Tiefschwarze Kosmetikminenformulierung für Applikationen am Auge. Mine im Durchmesser von 3 mm, für die Applikation auf trockener und auch feuchter Haut (Eyeliner- und Kajal-Anwendung). Die Minen sind bis 60°C applizierbar und besitzen eine Temperaturstabilität von mehr als 95°C.

| | |
|---|---|
| Aluminiumdistearat (CAS 300-92-5): | 4,0 % |
| Caprylic/Capric Triglyceride: (Handelsname Myritol 318; Lieferant/Hersteller: Henkel) | 16,0 % |
| Pigment Blue 29 CI 77007 | 5,0 % |
| Glimmer mit Siliciumdioxid beschichtet (Handelsname: Micronasphere M (Merck) | 15,0 % |
| Pigment Black 11; CI 77499 | 60,0 % |

### Beispiel 3:

Kreide im Durchmesser 10 mm für metallisch glänzende Hautmarkierungen mit einer maximalen Applikationstemperatur von 55°C:

| | |
|---|---|
| Aluminiumstearat (CAS 7047-84-9) | 4,0 % |
| Zinkstearat (CAS 557-05-1): | 2,0 % |
| Hydrogenated Palm/Kernel Oil PEG-6-Ester: | 10,0 % |
| (Handelsname: Lipocire BP Pastilles; Hersteller/Lieferant: Gattefossé) | 10,0 % |
| Hydrogenated Coconut Oil (CAS 84836-98-6) | 12,0 % |
| Talkum: | 20,0 % |
| Aluminiumpulver: Pigment Metal 1; CI 77000 | 42,0 % |

### Beispiel 4:

Formulierung für eine tiefrote Lippenstiftmine 8,0 mm mit tiefrotem, nicht glänzenden Farbabgabe und einem Schmelzpunkt von > 90°C:

| | |
|---|---|
| Aluminiumdistearat: | 4,0 % |
| Stearyl Dimethicone (Handelsname: Dowcorning 2503) | 16,0 % |
| (Hersteller: Dowcorning) | |
| Propylparaben: | 1,0 % |
| Talkum: | 28,0 % |
| Natural Red 4; CI 75470 | 51,0 % |

### Beispiel 5:

Schwach färbende Minenformulierung mit Heilerde (Löss) für Applikationen auf der Haut. Mine mit Durchmesser von 5 mm, für die Applikation auf trockener und auch feuchter Haut. Die Minen sind bis 60°C applizierbar und besitzen eine Temperaturstabilität von mehr als 95°C.

| | |
|---|---|
| Aluminiumdistearat (CAS 300-92-5): | 4,0 % |
| Caprylic/Capric Triglyceride: (Handelsname Myritol 318; Lieferant/Hersteller: Henkel) | 16,0 % |
| Heilerde (Löss), gemahlen (D90 ≤ 20 µm) | 50 % |
| Hydrogenated Palmkernelglycerides (Handelsname Lipocire CM; Bezug bei: Gattefossé): | 5 % |
| Glimmer mit Siliciumdioxid beschichtet (Handelsname: Micronasphere M (Merck) | 24,0 % |
| Propylparaben: | 1,0 % |

Sämtliche Prozentangaben sind Gewichtsprozent.

## Patentansprüche

1. Kosmetikmine mit einer Minengrundmasse und darin verteilten Feststoffpartikeln
**gekennzeichnet durch**
eine von wenigstens einem Fettsäuresalz und wenigstens einer lipophilen Substanz mit einem Schmelzbereich < 50°C gebildeten Minengrundmasse und folgender Zusammensetzung:
| | |
|---|---|
| Fettsäuresalz: | 1 - 20 Gew.% |
| Lipophile Substanz | 5 - 40 Gew.% |
| Feststoffpartikel: | 50 - 90 Gew.% |
| Additive: | 0 - 10 Gew.% |

2. Kosmetikmine nach Anspruch 1,
**dadurch gekennzeichnet,**
**dass** Fettsäuresalz mit einem Anteil von 3 Gew.% bis 10 Gew.% enthalten ist.

3. Kosmetikmine nach Anspruch 1,
**dadurch gekennzeichnet,**
**dass** Fettsäuresalz mit einem Anteil von 4 Gew.% bis 8 Gew.% enthalten ist.

4. Kosmetikmine nach einem der vorhergehenden Ansprüche,
**dadurch gekennzeichnet,**
**dass** die lipophile Substanz mit einem Anteil von 10 Gew.% bis 35 Gew.% enthalten ist.

5. Kosmetikmine nach einem der vorhergehenden Ansprüche,
**dadurch gekennzeichnet,**
**dass** die lipophile Substanz mit einem Anteil von 16 Gew.% bis 32 Gew.% enthalten ist.

6. Kosmetikmine einem der vorhergehenden Ansprüche,
**dadurch gekennzeichnet,**
**dass** mindestens 60 Gew.% bis 80 Gew.% Feststoffpartikel enthalten sind

7. Kosmetikmine nach einem der vorhergehenden Ansprüche,
**gekennzeichnet durch**
Fettsäuresalze mit 12 bis 18 C-Atomen.

8. Kosmetikmine nach Anspruch 7,
**gekennzeichnet durch**
wenigstens ein Fettsäuresalz aus der Gruppe, Calciumlauriat, Magnesiumstearat, Zinkstearat, sowie Salze der Palmitinsäure, der Linolsäure, der Ölsäure und der Myristinsäure.

9. Kosmetikmine nach Anspruch 7,
**gekennzeichnet durch**
wenigstens ein Fettsäuresalz aus der Gruppe Lithiumstearat, Aluminiumstearat und Aluminiumtristearat.

10. Kosmetikmine nach Anspruch 7,
**dadurch gekennzeichnet,**
**dass** als Fettsäuresalz Aluminiumdistearat enthalten ist.

11. Kosmetikmine nach einem der vorhergehenden Ansprüche,
**gekennzeichnet durch**
wenigstens eine lipophile Substanz aus der Gruppe Mineralöl, Castoröl, Isopropylmyristat, Isopropylpalmitrat, hydrogeniertes Kokosöl, Stearyl-dimethicone, Capryl-Glyceride, Caprin-Glyceride, Hydrogenierte Palmkernöl-Glyceride und Hydrogenierte Palmöl-Glyceride.

12. Kosmetikmine nach einem der vorhergehenden Ansprüche,
**dadurch gekennzeichnet,**
**dass** als Feststoffpartikel anorganische und/oder organische Farbpigmente enthalten sind.

13. Kosmetikmine nach Anspruch 1,
**dadurch gekennzeichnet**
**dass** Feststoffpartikel aus der Gruppe Talkum, Kaolin, Glimmer, mit Siliciumdioxid beschichteter Glimmer enthalten sind.

14. Kosmetikmine nach einem der vorhergehenden Ansprüche,
**gekennzeichnet durch**
wenigstens ein Additiv aus der Gruppe Aromen, Pflegesubstanzen, Filmbildner, Konservierungsmittel, Sonnenschutzmittel, Parfums, Vitamine und pflegende Substanzen.

15. Verfahren zur Herstellung einer Kosmetikmine nach einem der vorhergehenden Ansprüche, bei dem eine durch Mischung der Inhaltsstoffe der Kosmetikmine erhaltene Ausgangsmasse bei 80°C - 140°C extrudiert wird.

## Claims

1. Cosmetic lead with a lead basic mass and solids particles distributed therein
**characterized by**
a lead basic mass formed from at least one fatty acid salt and at least one lipophilic substance with a melting range < 50°C and of the following composition:
| | |
|---|---|
| Fatty acid salt: | 1-20% by wt. |
| Lipophilic substance: | 5-40% by wt. |
| Solids particles: | 50-90% by wt. |
| Additives: | 0-10% by wt. |

2. Cosmetic lead according to Claim 1,
**characterized in that**
the fatty acid salt is present in an amount of from 3% by weight to 10% by weight.

3. Cosmetic lead according to Claim 1,
**characterized in that**
the fatty acid salt is present in an amount of from 4% by weight to 8% by weight.

4. Cosmetic lead according to one of the preceding claims,
**characterized in that**
the lipophilic substance is present in an amount of from 10% by weight to 35% by weight.

5. Cosmetic lead according to one of the preceding claims,
**characterized in that**
the lipophilic substance is present in an amount of from 16% by weight to 32% by weight.

6. Cosmetic lead according to one of the preceding claims,
**characterized in that**
at least 60% by weight to 80% by weight of solids particles are present.

7. Cosmetic lead according to one of the preceding claims,
**characterized by**
fatty acid salts having 12 to 18 carbon atoms.

8. Cosmetic lead according to Claim 7,
**characterized by**
at least one fatty acid salt from the group calcium laurate, magnesium stearate, zinc stearate, and salts of palmitic acid, of linoleic acid, of oleic acid and of myristic acid.

9. Cosmetic lead according to Claim 7,
**characterized by**
at least one fatty acid salt from the group lithium stearate, aluminium stearate and aluminium tristearate.

10. Cosmetic lead according to Claim 7,
**characterized in that**
aluminium distearate is present as fatty acid salt.

11. Cosmetic lead according to one of the preceding claims,
**characterized by**
at least one lipophilic substance from the group mineral oil, castor oil, isopropyl myristate, isopropyl palmitate, hydrogenated coconut oil, stearyl dimethicones, caprylic glycerides, capric glycerides, hydrogenated palm kernel oil glycerides and hydrogenated palm oil glycerides.

12. Cosmetic lead according to one of the preceding claims,
**characterized in that**
inorganic and/or organic colour pigments are present as solids particles.

13. Cosmetic lead according to Claim 1,
**characterized in that**
solids particles from the group talc, kaolin, mica, mica coated with silicon dioxide are present.

14. Cosmetic lead according to one of the preceding claims,
**characterized by**
at least one additive from the group aromas, care substances, film formers, preservatives, sunscreens, perfumes, vitamins and caring substances.

15. Method of producing a cosmetic lead according to one of the preceding claims, in which a starting mass obtained by mixing the ingredients of the cosmetic lead is extruded at 80°C - 140°C.

## Revendications

1. Mine cosmétique ayant une composition de base et des particules de matières solides qui y sont réparties
**caractérisée par**
une composition de base formée d'au moins un sel d'acide gras et d'au moins une substance lipophile ayant un domaine de fusion <50°C et ayant la composition suivante :
| | |
|---|---|
| Sel d'acide gras | : de 1 à 20 % en poids |
| Substance lipophile | : de 5 à 40 % en poids |
| Particules de matières solides | : de 50 à 90 % en poids |
| Additifs | : de 0 à 10 % en poids. |

2. Mine cosmétique suivant la revendication 1,
**caractérisée**
**en ce que** le sel d'acide gras est contenu en une proportion de 3 % en poids à 10 % en poids.

3. Mine cosmétique suivant la revendication 1,
**caractérisée**
**en ce que** le sel d'acide gras est contenu en une proportion de 4 % en poids à 8 % en poids.

4. Mine cosmétique suivant l'une des revendications précédentes,
**caractérisée**
**en ce que** la substance lipophile est contenue en une proportion de 10 % en poids à 35 % en poids.

5. Mine cosmétique suivant l'une des revendications précédentes,
**caractérisée**
**en ce que** la substance lipophile est contenue en une proportion de 16 % en poids à 32 % en poids.

6. Mine cosmétique suivant l'une des revendications précédentes,
**caractérisée**
**en ce qu'**elle contient d'au moins 60 % en poids à 80 % en poids de particules de matières solides.

7. Mine cosmétique suivant l'une des revendications précédentes,
**caractérisée**
**en ce que** le sel d'acide gras a de 12 à 18 atomes de carbone.

8. Mine cosmétique suivant la revendication 7,
**caractérisée**
**par** au moins un sel d'acide gras choisi dans le groupe laurate de calcium, stéarate de magnésium, stéarate de zinc, sel de l'acide palmitique, de l'acide linoléique, de l'acide oleique et de l'acide myristique.

9. Mine cosmétique suivant la revendication 7,
**caractérisée**
**en ce qu'**au moins un sel d'acide gras est choisi dans le groupe stéarate de lithium, stéarate d'aluminium et tristéarate d'aluminium.

10. Mine cosmétique suivant la revendication 7,
**caractérisée**
**en ce que** du distéarate d'aluminium est contenu en tant que sel d'acide gras.

11. Mine cosmétique suivant l'une des revendications précédentes,
**caractérisée par**
au moins une substance lipophile choisie dans le groupe d'une huile minérale, d'une huile de ricin, du myristate d'isopropyle, du palmitate d'isopropyle, d'huile de coprah, de stéaryl-diméthicone, de glycéride de capryle, de glycéride de caprine, de glycéride d'huile de palmiste hydrogénée et de glycéride d'huile de palme hydrogénée.

12. Mine cosmétique suivant l'une des revendications précédentes,
**caractérisée**
**en ce que** des pigments colorés minéraux et/ou organiques sont contenus en tant que particules de matières solides.

13. Mine cosmétique suivant la revendication 1, **caractérisée**
**en ce que** des particules de matières solides choisies dans le groupe du talc, du kaolin, du mica, de mica revêtu de dioxyde de silicium, sont contenues.

14. Mine cosmétique suivant l'une des revendications précédentes,
**caractérisée**
**par** au moins un additif choisi dans le groupe des agents aromatisants, des substances de soin, des agents filmogènes, des agents de conservation, des agents de protection vis-à-vis du soleil, des parfums, des vitamines et des substances de soin.

15. Procédé de fabrication d'une mine cosmétique suivant l'une des revendications précédentes, dans lequel on extrude entre 80°C et 140°C une composition de départ obtenue par mélange des substances contenues dans la mine cosmétique.
